# FASCICULE DE BREVET EUROPEEN

(11) **EP 1 497 417 B1**
(45) Date de publication et mention de la délivrance du brevet: **14.03.2007**
(21) Numéro de dépôt: 03740654.3
(22) Date de dépôt: 23.04.2003
(51) Int. Cl.: C12N 9/12, A61K 38/04, A61K 39/00, C12N 15/11, C12N 15/62

(54) **EPITOPES T DE L'ANTIGENE EPHA2**
EPITOPEN AUS DEM EPHA2-ANTIGEN
EPHA2 ANTIGEN T EPITOPES

(30) Priorité: 23.04.2002 FR 0205048
(43) Date de publication de la demande: 19.01.2005
(73) Titulaire: INSTITUT NATIONAL DE LA SANTE ET DE LA RECHERCHE MEDICALE (INSERM), 75013 Paris (FR); Institut Gustave Roussy, 94800 Villejuif (FR)
(72) Inventeur: KOSMATOPOULOS, Kostas, F-75013 PARIS (FR); ALVES, Pedro, F-92190 MEUDON (FR)
(74) Mandataire: Vialle-Presles, Marie José
(86) Numéro de dépôt international: PCT/FR2003/001280
(87) Numéro de publication internationale: WO 2003/091383

(56) Documents cités:
- US-A1- 2002 025 570
- US-B1- 6 323 016
- DATABASE CA [en ligne] CHEMICAL ABSTRACTS SERVICE, COLUMBUS, OHIO, US; HIROAKI, HIDEKAZU ET AL: "Determination of the solution structure of the SH3 domain of human p56 Lc tyrosine kinase" retrieved from STN Database accession no. 126:16202 CA XP002234125 & JOURNAL OF BIOMOLECULAR NMR (1996), 8(2), 105-122, 1996,
- R A LINDBERG & T HUNTER: "cDNA cloning and characterization of eck, an epithelial cell receptor protein-tyrosine kinase in the eph/elk family of protein kinases" MOLECULAR AND CELLULAR BIOLOGY., vol. 10, no. 12, décembre 1990 (1990-12), pages 6316-6324, XP000615509 AMERICAN SOCIETY FOR MICROBIOLOGY, WASHINGTON., US ISSN: 0270-7306 cité dans la demande

## Description

La présente invention est relative à des peptides dérivés de la protéine EphA2 et à leur utilisation en immunothérapie antitumorale.

La vaccination ou immunothérapie peptidique est une approche thérapeutique qui fait actuellement l'objet d'un grand intérêt dans le cadre de la prévention ou du traitement des cancers. Son principe repose sur l'immunisation par des peptides reproduisant des épitopes T d'antigènes tumoraux reconnus par les lymphocytes T cytotoxiques (CTL), qui jouent un rôle majeur dans l'élimination des cellules cancéreuses exprimant ces antigènes à leur surface.

On rappellera que les CTL ne reconnaissent pas les antigènes protéiques entiers, mais des fragments peptidiques de ceux-ci, présentés par les molécules du complexe majeur d'histocompatibilité (CMH) exprimées à la surface de différentes cellules. Ce sont ces fragments peptidiques qui constituent les épitopes T.

La présentation de ces peptides résulte d'un processus complexe, dénommé « apprêtement de l'antigène », qui implique 3 étapes principales :
- la dégradation cytosolique des antigènes par un complexe multienzymatique dénommé protéasome ;
- la translocation des peptides issus de cette dégradation dans le réticulum endoplasmique (RE) par les transporteurs TAP ;
- l'association de ces peptides avec le CMH pour former des complexes stables peptide/CMH, qui seront exportés à la surface cellulaire.

La présentation des épitopes T à la surface cellulaire dépend notamment de la stabilité de la protéine antigénique dans le cytosol, des sites et de la fréquence des coupures effectuées par le protéasome, de l'efficacité de la translocation dans le RE par les transporteurs TAP, et de la capacité des peptides à se fixer aux molécules du CMH et à former des complexes peptide/CMH stables.

Les épitopes présentés par le complexe majeur d'histocompatibilité de classe I (CMH I) ont généralement 8 à 11 acides aminés, et sont reconnus par les cellules T CD8+, qui représentent la composante majeure de la réponse cytotoxique. Les épitopes présentés par le complexe majeur d'histocompatibilité de classe II (CMH II) ont généralement 13 à 18 acides aminés et sont reconnus par les cellules T CD4+.

L'identification de ces épitopes, et notamment (compte tenu du rôle essentiel de la réponse CD8+ dans la cytotoxicité) de ceux présentés par le CMH I, constitue donc une étape essentielle pour le développement de compositions d'immunothérapie anti-tumorale.

De nombreux antigènes tumoraux capables d'induire une réponse CTL sont connus à l'heure actuelle. Certains des épitopes T de ces antigènes ont été identifiés, et l'efficacité de vaccins à base de peptides reproduisant ces épitopes T a été montrée dans de nombreux cas. Cependant, l'expression de la majorité de ces antigènes est restreinte à certains types histologiques de tumeurs, ce qui limite leur utilisation clinique. Il est donc souhaitable d'identifier d'autres antigènes tumoraux exprimés par un grand nombre de tumeurs d'origine variée, et qui soient en outre capables d'induire une réponse immunitaire cytotoxique antitumorale.

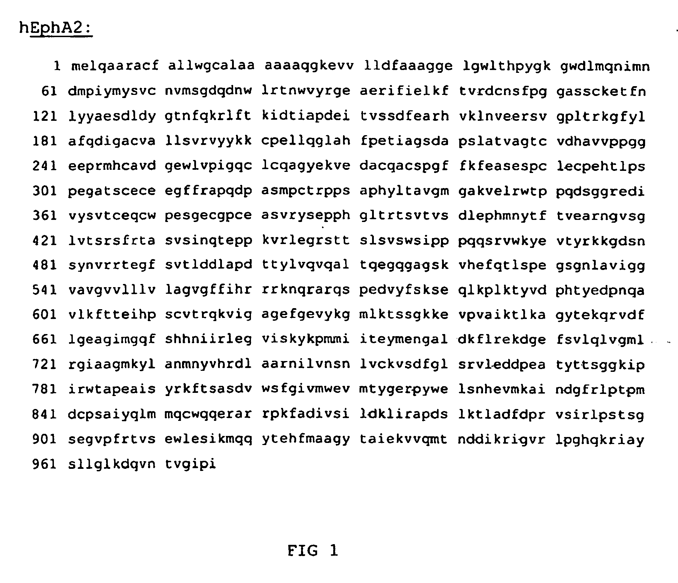

Le récepteur EphA2, précédemment dénommé ECK (LINDBERG et HUNTER, Molec. Cell. Biol. 10, 6316-6324, 1990), est un récepteur membranaire, possédant une activité tyrosine kinase. La séquence du récepteur EphA2 humain est représentée dans la figure 1 (SEQ ID NO : 1). Ce récepteur comprend un domaine extracellulaire de 534 acides aminés, un domaine transmembranaire de 24 acides aminés, et un domaine cytoplasmique de 418 acides aminés qui contient le domaine tyrosine kinase. Ce récepteur est surexprimé dans plusieurs types de cancer tels que le cancer du côlon, du sein, de la prostate, du poumon, de l'estomac, de l'oesophage ainsi que le mélanome métastatique, mais n'est pas surexprimé dans des lésions non cancéreuses de ces mêmes tissus (ROSENBERG *et al.* Am. J. Physiol. 273, 824, 1997; ZELINSKI *et al.* Cancer Res. 61, 2301, 2001; NEMOTO *et al.* Pathobiology 65, 195, 1997, EASTY *et al.* Int. J. Cancer 60, 129, 1995; WALKER DANIEL et al. Prostate 41, 275, 1999). Il a été observé que la surexpression d'EphA2 était liée à la transformation maligne et facilitait la progression métastatique des tumeurs. De plus, EphA2 joue un rôle important dans la néovascularisation tumorale (OGAWA et al. Oncogene 19, 6043, 2000).

Du fait de sa surexpression dans de nombreux types de tumeurs, et de son implication dans la transformation maligne et dans l'angiogenèse tumorale, il a été proposé d'utiliser EphA2 comme cible de traitements antitumoraux. Ainsi, la Demande PCT WO 01/12172 propose l'utilisation d'anticorps dirigés contre des épitopes B portés par le domaine extracellulaire du récepteur EphA2 pour l'immunothérapie antitumorale passive.

Cependant, on ignorait jusqu'à présent si EphA2 pouvait être apprêté efficacement pour générer des épitopes T capables d'induire une réponse cytotoxique antitumorale. *A fortiori,* aucun épitope T de cet antigène n'avait été identifié.

Les Inventeurs ont maintenant identifié dans EphA2 des peptides immunogènes présentés par le CMH I, et induisant des lymphocytes T cytotoxiques capables de lyser des cellules tumorales exprimant EphA2.

La présente invention a en conséquence pour objet un peptide immunogène constituant un épitope T présenté par le CMH I, caractérisé en ce qu'il est constitué par un fragment de 8 à 11 acides aminés consécutifs de l'antigène EphA2.

Dans le cadre de l'exposé de la présente invention, on entend par « peptide immunogène » un peptide capable d'induire une réponse CTL spécifique contre l'antigène EphA2.

Des peptides conformes à l'invention peuvent être obtenus de diverses manières à partir de l'antigène EphA2. Par exemple il est connu que des peptides susceptibles de former un complexe avec une molécule du CMH I donnée ont en commun la présence, à certaines positions, de résidus d'acides aminés particuliers, dénommés « résidus d'ancrage ». On a ainsi défini pour les différentes molécules du CMH I, des motifs d'ancrage spécifiques, impliquant des acides aminés dénommés « résidus d'ancrage primaires ». Il a aussi été montré que des résidus situés en dehors des motifs d'ancrage primaires (résidus d'ancrage secondaires) pouvaient exercer un effet favorable ou défavorable sur l'affinité du peptide pour le CMH.

Le choix des séquences peptidiques susceptibles de constituer des épitopes présentés par une molécule du CMH I donnée, peut s'effectuer, de manière classique, par l'analyse de la séquence peptidique de l'antigène EphA2, afin de sélectionner les peptides possédant tout ou partie du motif d'ancrage primaire correspondant à cette molécule. Différentes bases de données répertoriant les motifs d'ancrage connus sont disponibles : à titre d'exemples, on citera la base SYFPEITHI (http://www.uni-tuebingen.de/uni/kxi/; RAMMENSEE et al., Immunogenetics, 50, 213-219, 1999), ou la base BIMAS (http://bimas.dcrt.nih.gov/molbio/hla bind ; PARKER *et al.,* J. Immunol. 152, 163, 1994).

Généralement, on déterminera ensuite l'affinité de liaison des peptides ainsi identifiés pour la molécule du CMH concernée, ainsi que la stabilité du complexe peptide/molécule du CMH I. En effet, les peptides non-immunogènes présentent le plus souvent une faible affinité pour les molécules du CMH I, et/ou forment avec celles-ci un complexe peu stable. Des méthodes pour déterminer l'affinité du peptide pour la molécule du CMH I, et la stabilité du complexe formé sont connues en elles-mêmes. On citera par exemple celle décrite par FIRAT *et al.* (Eur. J. Immunol., 29, 3112, 1999).

L'affinité d'un peptide pour une molécule du CMH I est le plus souvent définie par rapport à celle d'un peptide de référence (par exemple IVGAETFYV (SEQ ID NO :2) pour HLA-A*0201 ou RPHERNGFTV (SEQ ID NO :3) pour HLA-B*0702), sous forme d'affinité relative. L'affinité relative est définie comme le rapport entre la concentration du peptide testé et la concentration du peptide de référence permettant la formation dans les mêmes conditions, de la même quantité de complexe peptide/molécule du CMH I. Plus l'affinité relative est importante, plus l'affinité de liaison du peptide pour la molécule du CMH I sera faible.

La stabilité du complexe peptide/molécule du CMH I est souvent définie par la DC₅₀, qui représente le temps nécessaire à la dissociation de 50% des complexes formés.

Par exemple, dans le cas des peptides potentiellement immunogènes et présentés par HLA-A*0201, l'affinité relative est généralement inférieure à 5 et la DC₅₀ supérieure à 2 heures.

L'immunogénicité des peptides potentiellement immunogènes ainsi détectés peut être vérifiée, par exemple par des méthodes classiques de détermination de la capacité de ce peptide à générer, *in vivo, ex vivo,* ou *in vitro* une réponse CTL spécifique vis-à-vis de cellules-cibles chargées avec ce peptide, ou exprimant l'antigène EphA2 dont il est issu.

Les peptides possédant une faible affinité pour la molécule du CMH I concernée, et/ou formant avec cette dernière un complexe peu stable, ont généralement une faible immunogénicité. Toutefois, ces peptides peuvent présenter un intérêt thérapeutique, dans la mesure où il apparaît que les épitopes de faible affinité ne participent que peu ou pas à l'établissement de phénomènes de tolérance, qui constituent un des principaux écueils de la vaccination antitumorale.

Dans ce cas, il est possible de préparer des peptides variants dont l'immunogénicité est plus importante, par substitution d'un ou plusieurs des acides aminés du peptide natif par un ou plusieurs acides aminés favorables à l'affinité pour la molécule du CMH I concernée et/ou à la stabilité du complexe peptide/molécule du CMH I.

Ces peptides variants font également partie de l'objet de la présente invention.

Des acides aminés favorables à l'affinité pour une molécule du CMH I donnée et/ou à la stabilité du complexe peptide/molécule du CMH I, peuvent par exemple être constitués par des résidus d'ancrage, et notamment les résidus d'ancrage secondaires, connus pour la molécule du CMH I concernée. Ces résidus d'ancrage peuvent facilement être identifiés par consultation des bases de données disponibles, comme celles mentionnées ci-dessus.

A titre d'exemple de substitution permettant d'augmenter l'immunogénicité d'un peptide présenté par une molécule du CMH I, et notamment par HLA-A*0201, on citera la substitution de l'acide aminé N-terminal dudit peptide par une tyrosine, comme décrit dans la Demande PCT WO 02/08716.

L'affinité d'un peptide variant pour la molécule du CMH I concernée, ainsi que son immunogénicité peuvent ensuite être vérifiées comme indiqué ci-dessus pour les peptides natifs.

A titre d'exemple non limitatif de réalisation de la présente invention, les Inventeurs ont identifié cinq peptides, dénommés ci-après p58, p61, p546, p550 et p883, présentés par HLA-A*0201.

Les séquences (code 1 lettre) de ces peptides sont les suivantes :
p58 : IMNDMPIYM (SEQ ID NO: 4);
p61 : DMPIYMYSV (SEQ ID NO: 5);
p546 : VLLLVLAGV (SEQ ID.NO: 6);
p550 : VLAGVGFFI (SEQ ID NO: 7);
p883 : TLADFDPRV (SEQ ID NO: 8).

Ils ont également obtenu, à partir du peptide p61, qui ne présente qu'une faible affinité pour HLA-A*0201, et une faible immunogénicité, un peptide immunogène, dénommé ci-après p61Y de séquence YMPIYMYSV (SEQ ID NO: 9), résultant de la substitution du résidu N-terminal de p61 par un résidu tyrosine.

Ces peptides sont capables d'induire une réponse CTL spécifique vis-à-vis de cellules HLA-A*0201 exprimant EphA2. Ils induisent notamment une réponse cytotoxique vis-à-vis de cellules tumorales HLA-A*0201 issues de tumeurs de types variés.

La présente invention a également pour objet des compositions comprenant au moins un peptide immunogène conforme à l'invention, ou une molécule d'acide nucléique codant pour ledit peptide.

Il peut s'agir de compositions multiépitopiques, capables de générer une réponse CTL polyspécifique, et qui dans ce but comprennent également un ou plusieurs autre(s) épitope(s) immunogène(s). Ces autres épitopes peuvent être issus d'EphA2, ou d'un ou plusieurs autres antigènes.

Ces compositions multiépitopiques conformes à l'invention peuvent comprendre, pour être largement utilisables sur une population dont les individus portent des allèles HLA différents, des épitopes présentés par différentes molécules du CMH I. Elles peuvent également comprendre en outre au moins un épitope présenté par une molécule du CMH II, et capable d'induire une réponse T auxiliaire.

Selon un mode de réalisation préféré d'une composition conforme à l'invention, elle comprend au moins un polypeptide chimérique comprenant une ou plusieurs copies d'un peptide immunogène conforme à l'invention. Dans le cas d'une composition multiépitopique, ledit polypeptide chimérique comprend en outre une ou plusieurs copies d'au moins un autre épitope immunogène.

Un tel polypeptide chimérique peut être facilement obtenu par des méthodes connues en elles-mêmes, et notamment par les techniques classiques de l'ADN recombinant.

La présente invention a également pour objet les molécules d'acide nucléique codant pour un peptide immunogène ou pour un polypeptide chimérique conforme à l'invention.

La présente invention a également pour objet l'utilisation d'un épitope peptidique immunogène, d'une composition, ou d'une molécule d'acide nucléique conforme à l'invention pour l'obtention d'un médicament, et notamment d'un médicament destiné à l'immunothérapie antitumorale, et en particulier au traitement de tumeurs exprimant EphA2.

Ceci englobe une grande variété de tumeurs, parmi lesquelles on citera notamment les tumeurs du côlon, du sein, de la prostate, du poumon, de l'estomac, du rein, et de l'oesophage.

Les peptides p58, p61, p546, p550, p883 et p61Y sont notamment utilisables pour l'obtention de médicaments destinés au traitement de patients HLA-A*0201.

La présente invention englobe également les médicaments comprenant, en tant que principe actif, au moins un peptide immunogène, une composition, ou une molécule d'acide nucléique conforme à l'invention.

Selon un mode de réalisation préféré de la présente invention, lesdits médicaments sont des vaccins.

Des médicaments conformes à l'invention peuvent comprendre en outre les excipients usuels, ainsi que des adjuvants habituellement utilisés en immunothérapie, et permettant par exemple de favoriser l'administration du principe actif, de le stabiliser, d'augmenter son immunogénicité, etc.

La présente invention sera mieux comprise à l'aide du complément de description qui va suivre, qui se réfère à des exemples non-limitatifs illustrant l'induction d'une réponse cytotoxique antitumorale par des peptides conformes à l'invention issus de l'antigène EphA2.

### EXEMPLE 1 : IDENTIFICATION D'EPITOPES D'EPHA2 PRESENTES PAR LA MOLECULE HLA-A*0201.

La séquence d'acides aminés de la protéine EphA2 a été analysée à l'aide du logiciel BIMAS (*PARKER* et al., *J. Immunol. 152, 163, 1994)*, afin d'identifier des peptides potentiellement capables de se lier à HLA-A*0201. Parmi les épitopes potentiels identifiés, les cinq peptides suivants :
p58 : IMNDMPIYM (SEQ ID NO: 4);
p61 : DMPIYMYSV (SEQ ID NO: 5);
p546 : VLLLVLAGV (SEQ ID NO: 6);
p550 : VLAGVGFFI (SEQ ID NO: 7);
p883 : TLADFDPRV (SEQ ID NO: 8);
ont été sélectionnés.

Les peptides correspondant à ces séquences ont été synthétisés par SYNT:EM (Nîmes, France). La pureté (>85%) est contrôlée par chromatographie liquide à haut rendement en phase inverse. Les peptides sont lyophilisés, puis dissous dans du DMSO à 10 mg/mL et stockés à -80°C.

L'immunogénicité de ces peptides a été évaluée par la mesure de leur affinité pour HLA-A*0201. Celle-ci est définie par deux paramètres : l'affinité relative (RA) qui reflète la capacité des peptides à se fixer à HLA-A*0201, et la vitesse de dissociation des complexes HLA-A*0201/peptide (DC₅₀) qui témoigne de leur stabilité. Les peptides à affinité élevée (RA<5 et DC₅₀>2 hrs), sont potentiellement immunogènes, contrairement aux peptides à faible affinité (RA>5 et DC₅₀<2 hrs).

### Affinité relative :

Des cellules T2 (FIRAT *et al.,* Eur. J. Immunol., 29, 3112, 1999) (3x10⁵ cellules/mL) humaines, qui sont déficientes en transporteurs TAP, sont incubées à 37°C pendant 16 heures avec diverses concentrations (100 µM, 10 µM, 1 µM, 0,1 µM) de chaque peptide à tester dans du milieu RPMI 1640 sans sérum, supplémenté avec 100 ng/mL de β2-microglobuline humaine. Ensuite, elles sont lavées deux fois, et marquées avec l'anticorps monoclonal BB7.2 (PARHAM *et al.,* Hum. Immunol., 3, 4, 277-299, 1981) qui est spécifique de la molécule HLA-A*0201, puis avec un anticorps de chèvre anti-Ig de souris, couplé à l'isothiocyanate de fluorescéine (FITC).

Les cellules sont ensuite analysées en cytométrie de flux. Pour chaque concentration de peptide, la fluorescence spécifique de HLA-A*0201 est calculée en tant que pourcentage de la fluorescence obtenue avec 100 µM d'un peptide de référence (HIVpol 589 ; IVGAETEYV , SEQ ID NO: 2). L'affinité relative (RA) est définie comme le rapport de la concentration de chaque peptide induisant 20% de la fluorescence obtenue avec 100 µM du peptide de référence, à la concentration du peptide de référence induisant 20% de la fluorescence obtenue avec 100 µM dudit peptide de référence. Plus l'affinité relative est faible, et plus fortement le peptide se lie à HLA-A*0201. La RA moyenne pour chaque peptide est déterminée à partir d'au moins trois expériences indépendantes. Dans toutes les expériences, 20% de la fluorescence maximale ont été obtenus pour 1 à 3 µM du peptide de référence.

### Stabilité :

Des cellules T2 (10⁶/mL) sont incubées pendant une nuit à 37°C avec 100 µM de chaque peptide à tester dans du milieu RPMI 1640 sans sérum, supplémenté avec 100 ng/mL de β2-microglobuline humaine. Ensuite, elles sont lavées à quatre reprises pour éliminer les peptides libres, incubées avec du BREFELDIN A (SIGMA ; 10 µg/mL) pendant une heure pour prévenir l'expression à leur surface des molécules HLA-A*0201 nouvellement synthétisées, lavées et incubées à 37°C pendant 0, 2, 4, 6 ou 8 heures en présence de BREFELDIN A (0,5 µg/mL). Pour chaque temps d'incubation, les cellules sont ensuite marquées, comme indiqué ci-dessus, avec l'anticorps BB7.2, et analysées en cytométrie de flux pour évaluer la quantité de complexe peptide/HLA-A*0201 présent à leur surface. Cette quantité est évaluée par la formule : (fluorescence moyenne des cellules T2 préincubées avec le peptide - fluorescence moyenne des cellules T2 traitées dans des conditions similaires en l'absence de peptide). Le DC₅₀ (complexe de dissociation : DC) est défini comme étant le temps (en heures) requis pour la perte de 50% des complexes HLA-A*0201/peptide stabilisés à t=0.

Les résultats de ces expérimentations sont présentés dans le Tableau I ci-après.

**Tableau I**

| Peptide | Séquence | RA | DC₅₀ |
|---|---|---|---|
| p58 | IMNDMPIYM | 1 | 4 |
| p61 | DMPIYMYSV | >10 | ND |
| p61Y | YMPIYMYSV | 1,5 | ND |
| p546 | VLLLVLAGV | 1,4 | 4-6 |
| p550 | VLAGVGFFI | 1 | 4-6 |
| p883 | TLADFDPRV | 2,2 | 2-4 |

| | | | |
|---|---|---|---|
| ND : non déterminé. | | | |

Ces résultats montrent que les peptides p58, p546, p550 et p883 possèdent une affinité de liaison importante (RA de 1 à 2,2). En revanche, le peptide p61 présente une faible affinité pour HLA-A*0201 (RA > 10) et devrait donc ne pas être immunogène. Pour améliorer l'affinité de ce peptide pour HLA-A*0201, les inventeurs ont substitué l'acide aspartique en position 1 par une résidu tyrosine. Le peptide variant p61Y obtenu présente une affinité de liaison importante (RA=1,5), bien supérieure à celle du peptide dont il est dérivé.

Les résultats montrent également que les peptides p58, p546, p550 et p883 forment des complexes stables avec les molécules HLA-A*0201 (DC₅₀> 2h pour chacun d'entre eux).

### EXEMPLE 2 : IMMUNOGENICITE DES PEPTIDES P58, P61Y, P546, P550 ET P883 :

### Induction de CTL spécifiques par vaccination avec les peptides

L'immunogénicité des peptides p58, p61Y, p546, p550 et p883 a été évaluée par génération de CTL sur des souris transgéniques HHD (PASCOLO *et al.,* J. Exp. Med., 185, 2043, 1997). Ces souris sont β2m-/-,D^{b}-/- et expriment une monochaîne HLA-A*0201 composée des domaines α1 et α2 de HLA-A*0201 et des domaines α3 et intracellulaire de D^{b}, reliée par son extrémité N-terminale à l'extrémité C-terminale de la β2-microglobuline humaine par un peptide de 15 acides aminés.

Les souris HHD reçoivent une injection sous-cutanée à la base de la queue avec 100 µg de chaque peptide à tester émulsifié dans de l'adjuvant incomplet de Freund, en présence de 140 µg d'un épitope auxiliaire T dérivé de l'antigène « core » de HBV (128-140, séquence TPPAYRPPNAPIL,SEQ ID NO: 10).

Après 11 jours, des cellules spléniques prélevées sur les souris (5×10⁷ cellules dans 10 mL) sont stimulées *in vitro* avec le peptide à tester (10 µM). Au 6ème jour de culture, les populations qui répondent sont testées pour déterminer une cytotoxicité spécifique. Les cellules qui répondent sont restimulées *in vitro* à des intervalles d'une semaine avec 2×10⁷ cellules spléniques HHD irradiées (3000 rads) et 1 à 0,1 µM de peptide en présence de 50 UI/mL d'IL2 recombinante (PROLEUKIN, CHIRON CORP).

Des essais de cytotoxicité sont effectués 6 jours après la dernière stimulation.

Des cellules RMAS-HHD sont utilisées comme cibles pour étudier la cytotoxicité. Ces cellules sont obtenues par transfection de cellules RMAS murines avec la construction HHD comme décrit par PASCOLO *et al.* (J. Exp. Med., 185, 2043, 1997).

Ces cellules-cibles sont marquées avec 100 µCi de ⁵¹Cr pendant 90 minutes, puis lavées trois fois et étalées dans des plaques de 96 puits à fond rond (3×10³ cellules/puits dans 100 µl de RPMI 1640 + 3% de sérum de veau foetal). Elles sont chargées avec 1 µM du peptide à tester, ou d'un peptide témoin non-pertinent, à 37°C pendant 90 minutes.

Ensuite, 100 µl des cellules effectrices (rapport cellules effectrices/cellules cible = 40/1) sont ajoutés dans les puits et les plaques sont incubées à 37°C pendant 4 heures. Après incubation, 100 µl de surnageant sont collectés et la radioactivité est mesurée dans un compteur γ.

Le pourcentage de lyse spécifique est calculé par la formule : [(libération de ⁵¹Cr expérimentale-libération de ⁵¹Cr spontanée)/(libération de ⁵¹Cr maximale-libération de ⁵¹Cr spontanée)] × 100. Dans toutes les expériences, la libération spontanée est inférieure à 20% de la libération maximale induite par HCl 3N.

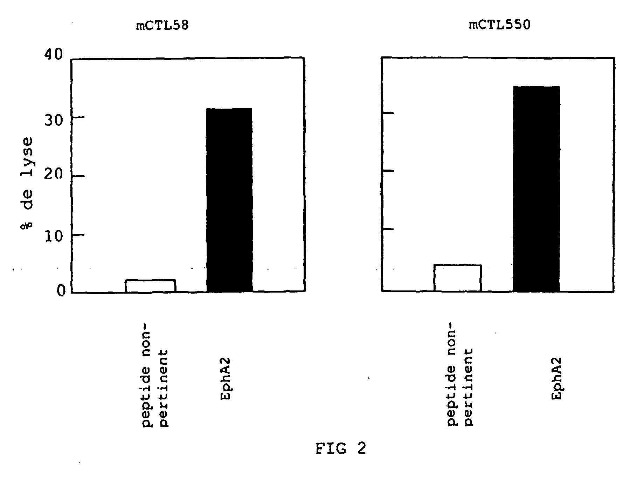

Les résultats de ces expérimentations pour les peptides p58 et p550 sont illustrés par la Figure 2.
□ : peptide non-pertinent ;
■ : peptide EphA2.

Ces résultats montrent que l'immunisation par le peptide p58 ou p550 génère des CTL qui tuent les cibles RMAS-HHD chargées avec ce même peptide, mais pas les cellules chargées avec le peptide non-pertinent. Des résultats équivalents ont été obtenus avec les peptides p61Y, p546 et p883.

Des lignées de CTL, respectivement dénommées mCTL58, mCTL61Y, mCTL546, mCTL550 et mCTL883 ont été établies, à partir des cellules spléniques de souris HDD immunisées avec le peptide p58, p61Y, p546, p550 ou p883, par stimulation répétées *in vitro* avec des concentrations décroissantes (10 µM-1 µM) du même peptide.

L'avidité de ces lignées pour leur peptide inducteur a été déterminée en mesurant, comme décrit ci-dessus, leur cytotoxicité vis-à-vis de cellules cibles RMAS-HHD chargées avec des concentrations croissantes (1 pM à 10 µM) du peptide concerné.

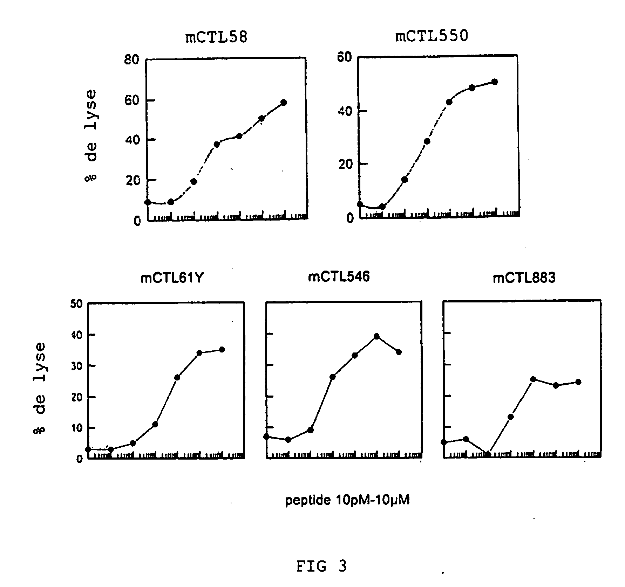

Les résultats sont illustrés par la Figure 3.

Ces résultats montrent que les lignées mCTL58, mCTL61Y, mCTL546, mCTL550 et mCTL883 possèdent une avidité relativement élevée. On obtient 50% de la lyse maximale pour des concentrations de peptide qui vont de 3 nM dans le cas de mCTL546 à 40 nM dans le cas de mCTL61Y.

### EXEMPLE 3 : RECONNAISSANCE DES EPITOPES APPRETES NATURELLEMENT DE L'ANTIGENE EPHA2 PAR DES CTL INDUITES PAR LES PEPTIDES P58 OU P550

Pour tester si les peptides p58 et p550 constituent des épitopes apprêtés naturellement de l'antigène EphA2, la réponse des cellules des lignées mCTL58 et mCTL550 à des cellules exprimant cet antigène a été évaluée de deux manières différentes.

### 1) Stimulation par des cellules COS-7 transfectées exprimant EphA2.

Les cellules des lignées mCTL58 et mCTL550, sont stimulées avec des cellules COS-7 de singe co-transfectées avec la construction HHD (PASCOLO *et al.* précité) et un plasmide contenant l'ADNc de EphA2. A titre de témoins négatifs on utilise des cellules COS-7 transfectées soit avec la construction HHD seule, soit avec le plasmide contenant l'ADNc de EphA2 seul.

La stimulation des CTL est évaluée par mesure de leur sécrétion de TNF-α. A titre de témoin positif, on utilise les cellules COS-7 transfectées avec la construction HHD et chargées avec le peptide p58 ou p550.

4 jours après la transfection, les cellules COS-7 sont mises en contact avec les cellules mCTL58 et mCTL550 à raison de 5×10⁴ CTL pour 3×10⁴ cellules COS-7 dans du RPMI 1640 en présence de 10% SVF.

Après 6 heures d'incubation, le surnageant est prélevé (50 µL), et mis en contact avec des cellules de fibrosarcome de souris WEHI164 clone 13 (3×10⁴ par puits) qui se caractérisent par une forte sensibilité à l'apoptose induite par le TNF-α. Afin de quantifier la teneur en TNF des surnageants de culture, une gamme étalon de TNF-α (concentrations de 0 à 10⁴ pg/mL) est utilisée en parallèle. Après 16 heures d'incubation à 37°C, la viabilité des cellules WEHI-164 clone 13 est déterminée par un test colorimétrique au MTT (SIGMA) (ESPEVIK et NISSEN MEYER, J. Immunol. Methods., 95, 99, 1986).

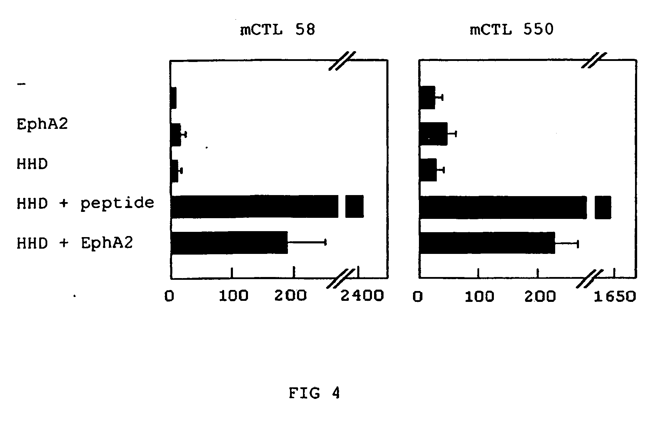

Les résultats sont illustrés par la Figure 4.
- : cellules COS-7 non transfectées ;
EphA2 : cellules COS-7 transfectées par l'ADNc d'EphA2 seul ;
HHD : cellules COS-7 transfectées par la construction HHD seule ;
HHD + peptide : cellules COS-7 transfectées avec la construction HHD et chargées avec le peptide p58 ou p550 ;
HHD+ EphA2 : cellules COS-7 transfectées avec la construction HHD et l'ADNc d'EphA2.

Ces résultats montrent que les lignées mCTL58 et mCTL550 répondent à la stimulation par les cellules COS co-exprimant HHD et EphA2.

En revanche, on n'observe aucune réponse aux cellules COS transfectées séparément par la construction HHD ou par l'ADNc d'EphA2.

### 2) Stimulation par des cellules tumorales humaines HLA-A*0201 exprimant EphA2.

Les lignées tumorales HLA-A*0201 suivantes ont été utilisées : SAOS (sarcome), 1355 (cancer du poumon), Caco-2 (cancer du côlon), HIEG (carcinome rénal), LNCaP (cancer de la prostate). La lignée DU145 (cancer de la prostate) n'exprimant pas HLA-A*0201 a également été utilisée à titre de témoin négatif.

Parmi ces lignées, DU145 et Caco-2 sont connues comme exprimant EphA2, et LNCaP comme n'exprimant pas EphA2.

L'expression d'EphA2 dans les autres lignées tumorales a été évaluée par transfert de Western. Le niveau d'expression EphA2 dans l'ensemble des lignées utilisées est résumé dans le Tableau II ci-dessous.

**Tableau II**

| Lignée cellulaire | Expression HLA-A*0201 | Expression EphA2 |
|---|---|---|
| SAOS | + | + |
| 1355 | + | + |
| Caco-2 | + | + |
| HIEG | + | + |
| LNCaP | + | - |
| DU145 | - | + |

| | | |
|---|---|---|
| + : forte expression -: pas d'expression. | | |

Les lignées mCTL58 et mCTL550 ont été stimulées par les lignées tumorales SAOS, 1355, Caco-2, HIEG, LNCaP, et DU145 mentionnées ci-dessus. Les lignées mCTL61Y, mCTL546 et mCTL 883 ont été stimulées par les lignées tumorales LNCaP, DU145 et Caco-2 mentionnées ci-dessus. La stimulation est évaluée par détection de la sécrétion de TNF-α, comme décrit ci-dessus.

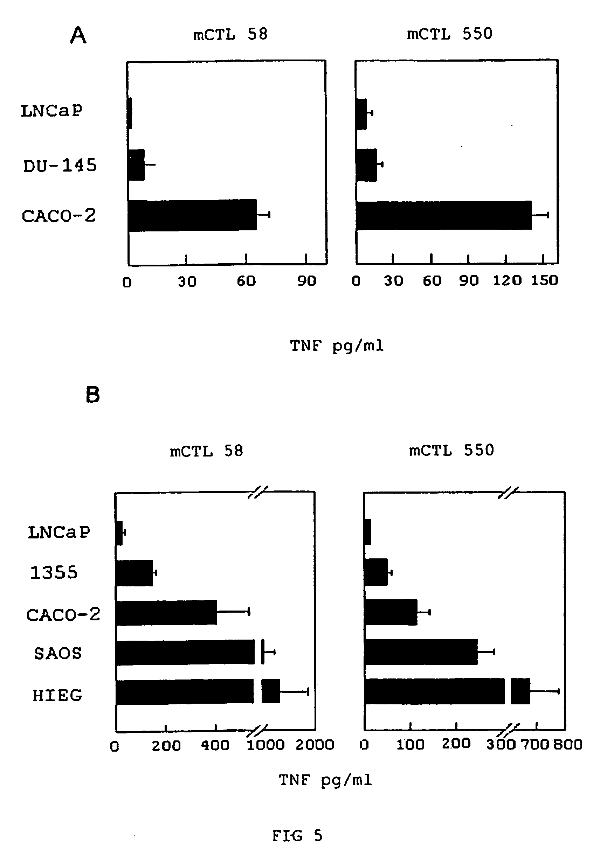

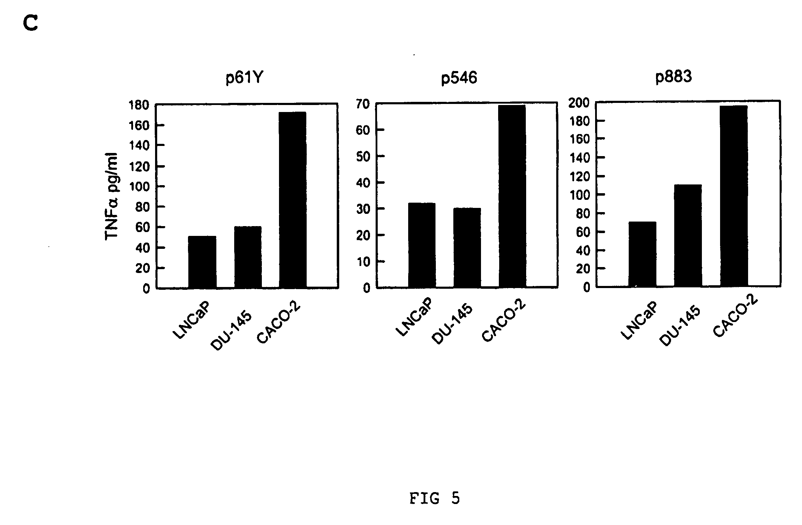

Les résultats sont illustrés par les Figures 5A, 5B et 5C.

La figure 5A montre que les cellules mCTL58 et mCTL550 répondent à la stimulation par les cellules Caco-2, qui expriment HLA-A*0201 et EphA2, mais ne répondent ni aux cellules DU145 qui n'expriment pas HLA-A*0201 ni aux cellules LNCaP qui n'expriment pas EphA2.

La figure 5B montre que les cellules mCTL58 et mCTL550 répondent à la stimulation par les cellules HIEG, Caco-2, 1355 et SAOS qui expriment des quantités importantes d'EphA2, mais ne répondent pas aux cellules LNCaP qui n'expriment pas EphA2.

La figure 5C montre que les cellules mCTL61Y, mCTL546 et mCTL883 répondent à la stimulation par les cellules Caco-2 qui expriment des quantités importantes d'EphA2, mais ne répondent pas aux cellules LNCaP et DU145 qui n'expriment pas EphA2 et HLA-A*0201 respectivement.

Les résultats des expérimentations ci-dessus montrent que les CTL induits par p58, p61Y, p546, p550 ou p883 reconnaissent des épitopes apprêtés naturellement de l'antigène EphA2.

### EXEMPLE 4 : INDUCTION DE CTL HUMAINS SPECIFIQUES DES PEPTIDES P58 OU P550.

La capacité de p58 et p550 à induire des CTL *in vitro* à partir de cellules mononucléées du sang périphérique (PMBC) de donneurs sains a été testée comme suit.

Les PBMC sont obtenues, à partir de prélèvements sanguins par leucocytaphérèse sur des donneurs sains, après centrifugation à 2000 rpm pendant 20 min sur gradient de Ficoll/Hypaque (AMERSHAM). Après 3 lavages en NaCl 0,9%, 10⁷ PBMC sont resuspendues dans chacun des puits d'une plaque de culture à 6 puits, dans 3 mL de milieu complet (RPMI 1640 supplémenté avec 10% de sérum humain AB inactivé par la chaleur), et incubées à 37°C pendant 2 heures. Après incubation, les cellules non-adhérentes sont prélevées et les cellules adhérentes sont différenciées en cellules dendritiques par ajout dans chacun des puits de 3 mL de milieu complet contenant 50 ng/mL de GM-CSF (R & D SYSTEMS) et 1000 UI/mL d'IL-4 (R & D SYSTEMS). Après 7 jours de culture les cellules dendritiques sont collectées et chargées avec le peptide p58 ou p550 par incubation pendant 4 heures à 20°C avec 40 µg/mL de peptide en présence de 3 µg/mL de β2-microglobuline, puis irradiées à 4200 rads : elles sont ensuite lavées pour éliminer le peptide libre. Des cellules CD8+ sont isolées à partir des cellules non-adhérentes à l'aide de microbilles couplées à un anticorps anti-CD8 (MILTENYI BIOTEC).

0,5×10⁶ cellules CD8+ sont stimulées par co-culture dans une plaque à 48 puits avec 2,5×10⁴ cellules dendritiques chargées avec le peptide p58 ou p550, dans du milieu complet supplémenté avec 10ng/mL d'IL-7 dans un volume final de 500 µl/puits. Le jour suivant la mise en culture, on ajoute dans chacun des puits 10 ng/mL d'IL-10 humaine (R & D SYSTEMS) ; le deuxième jour, on ajoute dans chacun des puits 30 UI/mL d'IL-2 humaine. Le septième et le quatorzième jour après la première stimulation, les cellules CD8+ sont restimulées avec les cellules adhérentes chargées par 10 µg/mL de peptide en présence de 3 µg/mL de β2-microglobuline et irradiées. De l'IL-10 (10 ng/mL) et de l'IL-2 (30 UI/mL) sont ajoutées respectivement 24 heures et 48 heures après restimulation. Sept jours après la seconde restimulation, la réponse de ces cellules à des cellules T2 chargées avec p58 ou p550 ou avec un peptide non-pertinent, ou à des cellules tumorales HLA-A*0201 Caco-2 (exprimant EphA2 et HLA-A*0201), LNCaP (exprimant HLA-A*0201 et n'exprimant pas EphA2), et DU145 (exprimant EphA2 et n'exprimant pas HLA-A*0201) est évaluée par dosage de la production d'IFNγ intra-cellulaire.

Les cellules hCTL58 ou hCTL550 sont incubées avec les cellules T2 chargées, ou avec les cellules de la lignée tumorale testée, en présence de 20 µg/mL de BREFELDINE-A (SIGMA). Après 6 heures, elles sont lavées, marquées avec un anticorps anti-CD8 conjugué à la r-phycoérythrine (CALTAG LABORATORIES) dans du PBS pendant 25 min à 4°C, lavées et fixées avec du paraformaldéhyde à 4%. Elles sont ensuite perméabilisées par de la saponine (SIGMA) à 0,2% dans du PBS, et marquées avec un anticorps monoclonal anti-IFNγ conjugué à l'allophycocyanine (PHARMINGEN).

Les cellules sont ensuite analysées en cytométrie de flux (FACSCalibur^{™} (BECTON DICKINSON) et logiciel CellQuest^{™}).

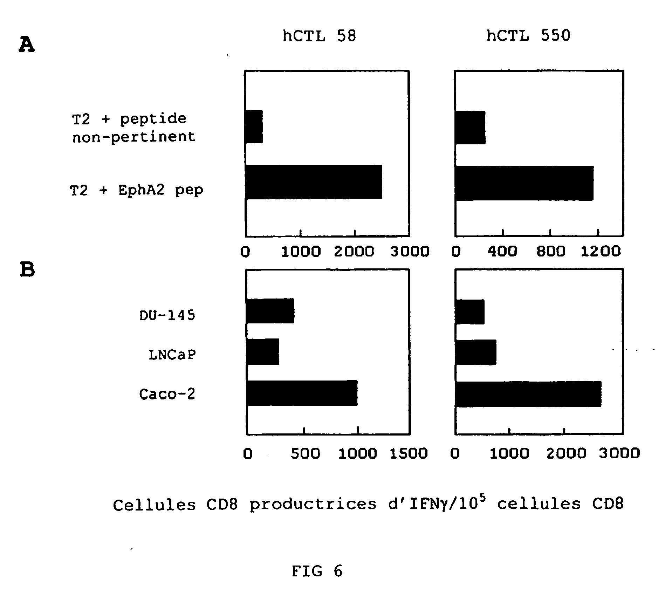

Les résultats (exprimés en nombre de cellules CD8+ productrices d'IFNγ pour 10⁵ cellules CD8+) sont illustrés par les Figures 6A et 6B.

La Figure 6A montre que les CTLs humains obtenus à partir de cellules CD8+ stimulées respectivement par le peptide p58 (hCTL58) ou le peptide p550 (hCTL550) sont activés par les cellules T2 chargées avec le peptide correspondant, et qu'on observe aucune activation par les cellules T2 chargées avec le peptide non-pertinent.

La Figure 6B montre une réponse des CTLs hCTL58 et hCTL550 vis-à-vis de la lignée tumorale Caco-2 (EphA2⁺, HLA-A*0201⁺), mais pas vis-à-vis des lignées LNCaP (EphA2⁻, HLA-A*0201⁺) et DU145 (EphA2⁺, HLA-A*0201⁻).

Ces résultats démontrent que les peptides p58 ou p550 induisent des CTLs humains capables de reconnaître des cellules tumorales HLA-A*0201+ exprimant EphA2.

## Revendications

1. Peptide immunogène constituant un épitope T présenté par le CMH I, **caractérisé en ce qu'**il est constitué par un fragment de 8 à 11 acides aminés consécutifs de l'antigène EphA2.

2. Peptide immunogène selon la revendication 1, **caractérisé en ce qu'**il est choisi parmi :
- le peptide de séquence IMNDMPIYM (SEQ ID NO:4);
- le peptide de séquence VLLLVLAGV (SEQ ID NO:6);
- le peptide de séquence VLAGVGFFI (SEQ ID NO:7);
- le peptide de séquence TLADFDPRV (SEQ ID NO:8).

3. Peptide immunogène **caractérisé en ce qu'**il est capable d'induire une réponse CTL spécifique contre l'antigène EphA2, et **en ce qu'**il est dérivé d'un peptide constitué par un fragment de 8 à 11 acides aminés consécutifs de l'antigène EphA2, par substitution d'au moins un acide aminé dudit peptide, par un acide aminé augmentant l'affinité dudit peptide pour une molécule du CMH I.

4. Peptide immunogène selon la revendication 3, **caractérisé en ce qu'**il est dérivé d'un peptide constitué par un fragment de 8 à 11 acides aminés consécutifs de l'antigène EphA2, par substitution de l'acide aminé N-terminal dudit peptide par un résidu tyrosine.

5. Peptide immunogène selon la revendication 4, **caractérisé en ce qu'**il est défini par la séquence YMPIYMYSV (SEQ ID No : 9).

6. Polynucléotide codant pour un peptide selon une quelconque des revendications 1 à 5.

7. Composition comprenant au moins un peptide selon une quelconque des revendications 1 à 5, ou un polynucléotide selon la revendication 6.

8. Composition selon la revendication 7, **caractérisée en ce qu'**il s'agit d'une composition multiépitopique comprenant en outre un ou plusieurs autre(s) peptide(s) immunogène(s) ou un ou plusieurs polynucléotide(s) codant pour le(s)dits peptide(s).

9. Composition selon la revendication 7, **caractérisé en ce qu'**il s'agit d'un polypeptide chimérique comprenant au moins une copie d'un peptide selon une quelconque des revendications 1 à 5 et au moins une copie d'un autre peptide immunogène, ou d'un polynucléotide codant pour ledit polypeptide chimérique.

10. Utilisation d'un peptide selon une quelconque des revendications 1 à 5, d'un polynucléotide selon la revendication 6, ou d'une composition selon une quelconque des revendications 7 à 9, pour l'obtention d'un médicament.

11. Utilisation selon la revendication 10, **caractérisée en ce que** ledit médicament est destiné à l'immunothérapie anti-tumorale.

12. Utilisation selon la revendication 11, **caractérisée en ce que** ledit médicament est destiné à l'immunothérapie de tumeurs exprimant l'antigène EphA2.

13. Utilisation selon une quelconque des revendications 10 à 12, **caractérisée en ce que** ledit médicament est destiné au traitement de patients HLA-A*0201.

## Claims

1. Immunogenic peptide constituting a T epitope presented by MHC I, **characterised in that** it is constituted by a fragment of from 8 to 11 consecutive amino acids of the EphA2 antigen.

2. Immunogenic peptide according to claim 1, **characterised in that** it is selected from:
- the peptide of sequence IMNDMPIYM (SEQ ID NO:4);
- the peptide of sequence VLLLVLAGV (SEQ ID NO:6);
- the peptide of sequence VLAGVGFFI (SEQ ID NO:7); and
- the peptide of sequence TLADFDPRV (SEQ ID NO:8).

3. Immunogenic peptide, **characterised in that** it is capable of inducing a specific CTL response against the EphA2 antigen, and **in that** it is derived from a peptide constituted by a fragment of from 8 to 11 consecutive amino acids of the EphA2 antigen by substitution of at least one amino acid of said peptide by an amino acid that increases the affinity of said peptide for a molecule of MHC I.

4. Immunogenic peptide according to claim 3, **characterised in that** it is derived from a peptide constituted by a fragment of from 8 to 11 consecutive amino acids of the EphA2 antigen by substitution of the N-terminal amino acid of said peptide by a tyrosine residue.

5. Immunogenic peptide according to claim 4, **characterised in that** it is defined by the sequence YMPIYMYSV (SEQ ID NO:9).

6. Polynucleotide coding for a peptide according to any one of claims 1 to 5.

7. Composition comprising at least one peptide according to any one of claims 1 to 5 or a polynucleotide according to claim 6.

8. Composition according to claim 7, **characterised in that** it is a multiepitope composition further comprising one or more other immunogenic peptide(s) or one or more polynucleotide(s) coding for said peptide(s).

9. Composition according to claim 7, **characterised in that** said peptide is a chimeric polypeptide comprising at least one copy of a peptide according to any one of claims 1 to 5 and at least one copy of another immunogenic peptide or a polynucleotide coding for said chimeric polypeptide.

10. Use of a peptide according to any one of claims 1 to 5 or of a polynucleotide according to claim 6 or of a composition according to any one of claims 7 to 9 in the preparation of a medicament.

11. Use according to claim 10, **characterised in that** said medicament is intended for antitumour immunotherapy.

12. Use according to claim 11, **characterised in that** said medicament is intended for the immunotherapy of tumours expressing the EphA2 antigen.

13. Use according to any one of claims 10 to 12, **characterised in that** said medicament is intended for the treatment of HLA-A*0201 patients.

## Patentansprüche

1. Immunogenes Peptid, das ein Epitop T bildet, das durch den MHC I präsentiert wird, **dadurch gekennzeichnet, dass** es durch ein Fragment mit 8 bis 11 fortlaufenden Aminosäuren des Antigens EphA2 gebildet wird.

2. Immunogenes Peptid nach Anspruch 1, **dadurch gekennzeichnet, dass** es ausgewählt ist aus:
- dem Peptid mit der Sequenz IMNDMPIYM (SEQ ID NO:4),
- dem Peptid mit der Sequenz VLLLVLAGV (SEQ ID NO:6),
- dem Peptid mit der Sequenz VLAGVGFFI (SEQ ID NO:7),
- dem Peptid mit der Sequenz TLADFDPRV (SEQ ID NO:8).

3. Immunogenes Peptid, **dadurch gekennzeichnet, dass** es fähig ist, eine spezifische CTL-Reaktion gegen das Antigen EphA2 zu induzieren und dass es von einem Peptid, das durch ein Fragment mit 8 bis 11 fortlaufenden Aminosäuren des Antigens EphA2 gebildet wird durch Substitution wenigstens einer Aminosäure des Peptids durch eine Aminosäure, die die Affinität des Peptids für ein Molekül des MHC I erhöht, abgeleitet ist.

4. Immunogenes Peptid nach Anspruch 3, **dadurch gekennzeichnet, dass** es von einem Peptid, das durch ein Fragment von 8 bis 11 fortlaufenden Aminosäuren des Antigens EphA2 gebildet wird, durch Substitution der N-terminalen Aminosäure des Peptids durch einen Tyrosinrest abgeleitet ist.

5. Immunogenes Peptid nach Anspruch 4, **dadurch gekennzeichnet, dass** es durch die Sequenz YMPIYMYSV (SEQ ID NO:9) definiert ist.

6. Polynukleotid, das für ein Peptid nach einem der Ansprüche 1 bis 5 codiert.

7. Zusammensetzung, umfassend wenigstens ein Peptid nach einem der Ansprüche 1 bis 5 oder ein Polynukleotid nach Anspruch 6.

8. Zusammensetzung nach Anspruch 7, **dadurch gekennzeichnet, dass** es sich um eine Multiepitop-Zusammensetzung handelt, umfassend außerdem ein anderes immunogenes Peptid oder mehrere andere immunogene Peptide oder ein Polynukleotid, das für das Peptid codiert, oder mehrere Polynukleotide, die für die Peptide codieren.

9. Zusammensetzung nach Anspruch 7, **dadurch gekennzeichnet, dass** es sich um ein chimäres Polypeptid handelt, das wenigstens eine Kopie eines Peptids nach einem der Ansprüche 1 bis 5 und wenigstens eine Kopie eines anderen immunogenen Peptids oder eines Polynukleotids, das für das chimäre Polypeptid codiert, umfasst.

10. Verwendung eines Peptids nach einem der Ansprüche 1 bis 5, eines Polynukleotids nach Anspruch 6 oder einer Zusammensetzung nach einem der Ansprüche 7 bis 9 für die Herstellung eines Medikaments.

11. Verwendung nach Anspruch 10, **dadurch gekennzeichnet, dass** das Medikament für die Antitumorimmuntherapie bestimmt ist.

12. Verwendung nach Anspruch 11, **dadurch gekennzeichnet, dass** das Medikament für die Immuntherapie von Tumoren bestimmt ist, die das Antigen EphA2 exprimieren.

13. Verwendung nach einem der Ansprüche 10 bis 12, **dadurch gekennzeichnet, dass** das Medikament zur Behandlung von HLA-A*0201-Patienten bestimmt ist.


### SEQUENCE LISTING

<110> INSERM
   IGR
   KOSMATOPOULOS, Kostas
   ALVES, Pédro
<120> EPITOPES T DE L'ANTIGENE EPHA2
<130> MJPah598/69EX
<160> 10
<170> PatentIn version 3.1
<210> 1
   <211> 976
   <212> PRT
   <213> Homo sapiens
<400> 1
<210> 2
   <211> 9
   <212> PRT
   <213> Human immunodeficiency virus
<400> 2
   Ile Val Gly Ala Glu Thr Phe Tyr Val
   1 5
<210> 3
   <211> 10
   <212> PRT
   <213> Human herpesvirus 5
<400> 3
   Arg Pro His Glu Arg Asn Gly Phe Thr Val
   1 5 10
<210> 4
   <211> 9
   <212> PRT
   <213> Homo sapiens
<400> 4
   Ile Met Asn Asp Met Pro Ile Tyr Met
   1 5
<210> 5
   <211> 9
   <212> PRT
   <213> Homo sapiens
<400> 5
   Asp Met Pro Ile Tyr Met Tyr Ser Val
   1 5
<210> 6
   <211> 9
   <212> PRT
   <213> Homo sapiens
<400> 6
   Val Leu Leu Leu Val Leu Ala Gly Val
   1 5
<210> 7
   <211> 9
   <212> PRT
   <213> Homo sapiens
<400> 7
   Val Leu Ala Gly Val Gly Phe Phe Ile
   1 5
<210> 8
   <211> 9
   <212> PRT
   <213> Homo sapiens
<400> 8
   Thr Leu Ala Asp Phe Asp Pro Arg Val
   1 5
<210> 9
   <211> 9
   <212> PRT
   <213> Artificial sequence
<220>
   <223> peptide immunogène
<400> 9
   Tyr Met Pro Ile Tyr Met Tyr Ser Val
   1 5
<210> 10
   <211> 13
   <212> PRT
   <213> Hepatitis B virus
<400> 10
   Thr Pro Pro Ala Tyr Arg Pro Pro Asn Ala Pro Ile Leu
   1               5                   10